# EUROPÄISCHE PATENTSCHRIFT

(11) **EP 0 436 761 B1**
(45) Veröffentlichungstag und Bekanntmachung des Hinweises auf die Patenterteilung: **02.11.1994**
(21) Anmeldenummer: 90100537.1
(22) Anmeldetag: 11.01.1990
(51) Int. Cl.: A61B 6/00

(54) **Röntgendiagnostikanlage**
X-ray diagnostic device
Appareil de radiodiagnostic

(43) Veröffentlichungstag der Anmeldung: 17.07.1991
(73) Patentinhaber: SIEMENS AKTIENGESELLSCHAFT, 80333 München (DE)
(72) Erfinder: Heinz, Lothar, Dipl.-Ing. (FH), D-8524 Neunkirchen (DE); Höbel, Peter, Dipl.-Ing. (FH), D-8520 Erlangen (DE); Pfeiler, Manfred, Dr.-Ing., D-8520 Erlangen (DE); Wessels, Gerd, Dr.-Ing., D-8520 Erlangen (DE)

(56) Entgegenhaltungen:
- DE-A- 2 855 379
- DE-B- 1 006 116
- US-A- 3 970 853

## Beschreibung

Die Erfindung betrifft eine Röntgendiagnostikanlage gemäß dem ersten Teil des Patentanspruches. Eine solche Röntgendiagnostikanlage ist beispielsweise durch die DE-A-10 06 116 oder die US-A-3 970 853 bekannt. Das Röntgengerät ist dabei ortsfest.

Wesentlich für die vorliegende Erfindung ist, daß ein fahrbares Röntgengerät vorhanden ist. Es ist bekannt, die Bildelektronik für ein fahrbares Röntgengerät, z.B. ein C-Bogengerät, in einem Wagen unterzubringen, welcher über eine feste Verkabelung mit dem Röntgengerät verbunden ist. Damit sich keine zu großen und störenden Kabellängen ergeben, muß die Bildelektronik immer in der Nähe des Röntgengerätes sein, was den Arzt bei der Untersuchung stören kann.

Der Erfindung liegt die Aufgabe zugrunde, eine Röntgendiagnostikanlage der eingangs genannten Art so auszubilden, daß die Bildelektronik getrennt vom Röntgengerät, insbesondere in einem getrennten Raum, angeordnet werden kann, ohne daß Kabel den Untersuchungsbetrieb stören.

Diese Aufgabe ist erfindungsgemäß gelöst durch die Merkmale des Patentanspruches.

Bei der erfindungsgemäßen Röntgendiagnostikanlage ist der Untersuchungsraum weitgehend frei von beim Verfahren des Röntgengerätes störenden Datenkabeln.

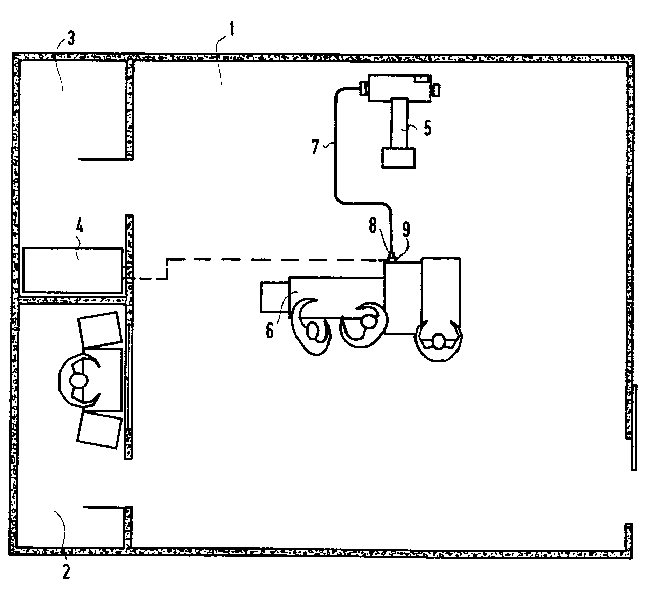

Die Erfindung ist nachfolgend anhand eines in der Zeichnung dargestellten Ausführungsbeispieles näher erläutert.

In der Zeichnung ist der Grundriß eines Untersuchungsraumes 1, eines Meß- und Kontrollraumes 2 und eines Rechnerraumes 3 mit einer Bildelektronik 4 dargestellt. Im Untersuchungsraum 1 ist ein fahrbares Röntgenuntersuchungsgerät 5, z.B. ein C-Bogengerät dargestellt, das für Röntgenuntersuchungen an einem Patienten auf einem Patientenlagerungstisch 6 dient. Das Röntgengerät 5 besitzt beispielsweise eine Bildverstärker-Fernsehkette zur Erzeugung von Bilddaten.

Die Bilddaten werden über ein Datenkabel 7 zu einem Datenstecker 8 übertragen, der in einer Datensteckdose 9 am Patientenlagerungstisch 6 steckt. Von der Datensteckdose 9 führt eine feste, unterirdische Verkabelung zur Bildelektronik 4. Durch Öffnen der Datenkupplung 8, 9 ist eine freie Verstellung des Röntgenuntersuchungsgerätes 5, z.B. in eine Parkstellung, möglich.

## Patentansprüche

1. Röntgendiagnostikanlage mit einem Röntgengerät (5) mit einer Bildaufnahmevorrichtung zur Erzeugung von Bilddaten und mit einer Datenübertragungsstrecke zur Übertragung der Bilddaten zu einer Bildelektronik (4), die vom Röntgengerät (5) getrennt angeordnet ist, wobei die Datenübertragungsstrecke eine Datenkupplung (8, 9) aufweist, deren einer Teil (8) dem Röntgengerät (5) und deren anderer Teil (9) der Bildelektronik zugeordnet ist, **dadurch gekennzeichnet,** daß das Röntgengerät (5) fahrbar ist und daß ein flexibles Datenkabel (7) vom Röntgengerät (5) zu einer Datensteckvorrichtung (9) an einem Patientenlagerungstisch (6) und von dort ein fest verlegtes Datenkabel zur Bildelektronik (4) führt.

## Claims

1. X-ray diagnostic installation having an X-ray unit (5) having an image pick-up arrangement for generating image data and having a data-transmission link for transmitting the image data to an image electronics unit (4) which is arranged separately from the X-ray unit (5), the data-transmission link having a data coupling (8, 9), the one portion (8) of which is associated with the X-ray unit (5) and the other portion (9) of which is associated with the image electronics unit, characterised in that the X-ray unit (5) is mobile and in that a flexible data cable (7) leads from the X-ray unit (5) to a data socket (9) on a patient-bearing table (6) and from there a fixedly installed data cable leads to the image electronics unit (4).

## Revendications

1. Installation de radiodiagnostic comportant un appareil radiologique (5) possédant un dispositif d'enregistrement d'images servant à former des données d'images et une section de transmission de données pour la transmission de données d'images à un système électronique (4) de traitement d'images, qui est disposé séparément de l'appareil radiologique (5), la section de transmission de données comportant une unité de couplage de données (8, 9), dont une partie (8) est associée à l'appareil radiologique (5) et dont l'autre partie (9) est associée au système électronique de traitement d'images, caractérisée par le fait que l'appareil radiologique (5) est mobile, qu'un câble flexible (7) de transmission de données relie l'appareil radiologique (5) à un dispositif d'enfichage (9) de transmission de données situé sur un plateau formant couchette pour patient (6), et qu'un câble de transmission de données s'étend à partir de là jusqu'au système électronique (4) de traitement d'images.
